# EUROPEAN PATENT SPECIFICATION

(11) **EP 3 407 715 B1**
(45) Date of publication and mention of the grant of the patent: **18.12.2019**
(21) Application number: 17714150.4
(22) Date of filing: 25.01.2017
(51) Int. Cl.: A01N 25/10, A01N 59/16, A01P 1/00, B05D 1/18, B82Y 30/00, B82Y 40/00, C08K 3/08, D01D 1/02, D01D 5/00, D01F 6/62, D01F 6/70, D01F 6/88, D04H 1/42, D04H 1/728, D06M 11/65, D06M 11/83, D06M 16/00, D06M 23/08, D06N 1/00, D01C 1/00

(54) **POLYMER SUBSTRATE WITH IMMOBILIZED SILVER NANOPARTICLES AND METHOD OF PREPARATION THEREOF**
POLYMERSUBSTRATE MIT IMMOBILISIERTEN SILBERNANOPARTIKELN UND VERFAHREN ZUR HERSTELLUNG DAVON
SUBSTRAT POLYMÈRE AVEC NANOPARTICULES D'ARGENT IMMOBILISÉES ET SON PROCÉDÉ DE PRÉPARATION

(30) Priority: 27.01.2016 CZ 20160038
(43) Date of publication of application: 05.12.2018
(73) Proprietor: Regionalni centrum pokrocilych technologii a materialu, Univerzita Palackeho v Olomouci, 78371 Olomouc (CZ)
(72) Inventor: SOUKUPOVA, Jana, 77900 Olomouc (CZ); ZBORIL, Radek, 77900 Olomouc (CZ)
(74) Representative: Hartvichova, Katerina
(86) International application number: PCT/CZ2017/050002
(87) International publication number: WO 2017/129153

(56) References cited:
- WO-A1-2013/029574
- WO-A1-2013/116408
- WO-A2-2013/023006
- KR-A- 20070 078 177
- KR-A- 20100 026 083
- US-A1- 2008 217 807

## Description

### Filed of Art

The present invention relates to a method of preparation of a polymer composite substrate with immobilized silver nanoparticles and to the substrate obtainable using the method.

### Background Art

Nowadays, there are several approaches to tailored immobilization of silver nanoparticles on various substrates (AgNPs) with the aim to provide antimicrobial treatment of the material or to provide antifouling protection (prolongation of lifetime of the filtration/fibrous structures). These approaches, however, do not solve the problem of potential nanoparticle release, potentially release of the silver nanoparticles with their linkers. The Czech patent CZ303502 describes the approach to immobilization of silver nanoparticles (AgNPs) via covalent bonds between the silver and the linker but it does not eliminate possible release of the whole composite, i.e. Ag-linker, from the substrate under mechanical stress or under high pressure of the flow and flow rate of the media - in the case of the filtration materials.

The above-described problem is solved by the present invention, which provides for the immobilization of silver nanoparticles onto the substrate in such a way that effectively prevents possible release of the silver nanoparticles/silver nanoparticles with a polymer linker from the composite substrate. This immobilization process represents rather simplified method as the functionalization of the surfaces can proceed under significantly lower temperatures. The temperature represents a crucial parameter especially from some substrates.

### Disclosure of the Invention

The present invention introduces a method of preparation of a polymer substrate with immobilized silver nanoparticles, wherein the substrate is prepared from a mixture of water non-soluble polymer and a polymer linker containing Ag-reducing functional groups (i.e. functional groups that are able to reduce Ag⁺ to Ag⁰) containing nitrogen atom with a free electron pair. Consequently, the substrate is functionalized with covalently bonded silver nanoparticles, which are generated from an aqueous solution of silver ions. The generated silver nanoparticles are covalently bonded to the substrate via the nitrogen atom and/or sulphur atom of the polymer linker.

Water non-soluble polymer is preferably a synthetic polymer, more preferably selected from a group comprising polyurethanes, polylactide, polyglycolides, poly(lactide-co-glycolide), polyethylene, polypropylene, polyamide, saccharides, as for example cellulose or chitosan.

The Ag-reducing functional groups are selected from the group comprising amino-, imino-, imido- functional groups. The polymer linker has preferably molecular weight from 300 g.mol⁻¹ to 1,000,000 g.mol⁻¹ and has either linear or branched structure. A preferable polymer linker is polyethylenimine.

The substrate is nanofibrous and is prepared from a water non-soluble polymer and a polymer linker using electrospinning method. It has been proved that if the substrate is prepared from a mixture of water non-soluble polymer and polymer linker, it is sufficient to prevent the undesired release of the composite of Ag-linker. The release of Ag-linker composite represented the disadvantage in CZ 303502.

Preferably, the polymer linker is involved in the substrate in the concentration from 0.0001 wt. % up to 5 wt.%. The concentration is related to the overall mass of the water non-soluble polymer.

Silver ions might be in a form of a compound containing silver ions, which can be a water soluble silver salt, as e.g. AgNO₃, AgClO₄, AgF, or a water soluble complex compound of silver ions, as e.g. diamminesilver complex.

Aqueous solution containing ionic silver reacts with the substrate, preferably in the concentration range of silver ions solution from 0.0001 mol·L⁻¹ up to 5 mol·L⁻¹; preferably at the temperature of from 5 °C to 95 °C, ideally at room temperature, preferably for the period from 1 s up to 60 minutes.

The resulting composite material exhibit the required antimicrobial properties thanks to the covalently bonded silver nanoparticles, which are even firmly anchored through the polymer linker, which itself is anchored into the structure of the substrate. In contrast to the method, when the silver nanoparticles are anchored into the substrate, in the method according to the present invention the overall surface of the silver nanoparticles is available for the antimicrobial action, and the silver nanoparticles do not release from the surface under mechanical stress, which represents a key parameter of such a composite for the use e.g. in the filtration/water treatment technologies or in medicinal applications.

The subject of the present invention is further an antimicrobial material containing silver nanoparticles, which structure is based on a substrate formed by a water non-soluble polymer and polymer linker containing a nitrogen atom and/or a sulphur atom onto which silver nanoparticles are covalently bonded. The antimicrobial material is prepared by using the method according to the present invention.

### Brief Description of Figures

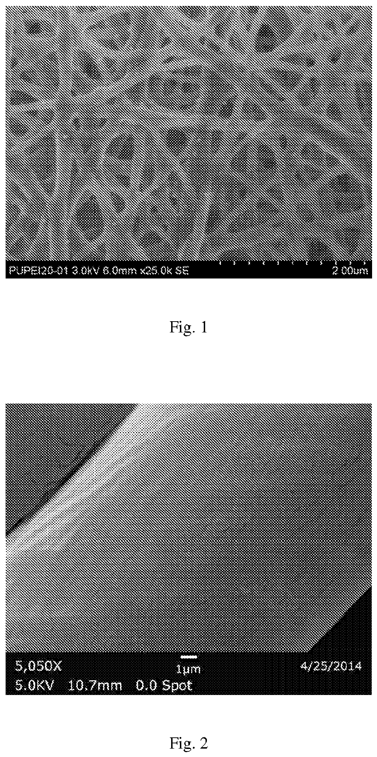

- Fig. 1: shows the SEM image of the nanofibers prepared according to the example 1.
- Fig. 2: shows the SEM image of the nanofibers prepared according to the example 2.

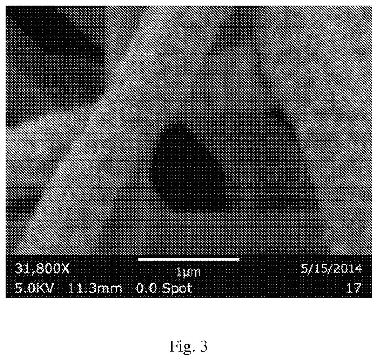

- Fig. 3: shows the SEM image of the nanofibers prepared according to the example 3.

### Examples of the Invention Embodiments

### Example 1: Polyurethane (PU) fibres with immobilized silver nanoparticles (on all surfaces)

Nanofibrous structures of polyurethane (PU), i.e. fibres with a diameter in the range from tens up to hundreds of nanometers, are prepared by electrospinning from the mixture of polyurethane dissolved in dimethylformamide (DMF). The weight percentage of PU dissolved in DMF is 4%. After homogenization of the DMF:PU mixture, the branched polyethylenimine (i.e. hyperbranched polymer linker containing nitrogen atoms with free electron pairs, which can be used for the reduction stabilization of subsequently generated and immobilized silver nanoparticles) was added. The molecular weight of the polyethylenimine was 25,000 g.mol⁻¹ (in the concentration of 1 wt.% to the PU mass). The homogenization of the mixture DMF:PU:PEI is followed by electrospinning of this mixture. The fibres are collected on a rotation collector as a compact layer of the mesh structured fibres (i.e. fibres without orientation). This structure contains fibres with immobilized polymer linker in its structure. Thanks to the immobilized linker the fibres can be preferably used for tailored deposition of silver nanoparticles when the fibres are immersed into the precursor of the silver nanoparticles solution, i.e. into silver nitrate with the concentration of 8·10⁻⁴ mol.L⁻¹. The mesh structure is taken out of the solution immediately and the structure is washed with water in order to get rid of the residues of the precursor from all the mesh cavities. The whole process is carried out under room temperature. There is a strong coordination-covalent interaction between the heteroatom and silver, and the linker itself is firmly anchored in the substrate structure. Based on the above-mentioned reasons the release of the silver nanoparticles or silver nanoparticles with the linker from the composite material is prevented.

The amount of silver in the prepared sample of PU fibres with immobilized AgNPs was consequently quantified with AAS. Comparable sample was placed into water and onto a shaker and let to be mechanically stressed. After 24 hours, the amount of silver on the composite material was quantified with AAS. The amount of silver on the tested (i.e. first sample) and stressed (i.e. the sample mechanically stressed for 24 hours in water on a shaker) sample was almost identical (note: the difference was 0.1 wt% of silver, which represents a statistical error can correspond with the released Ag⁺ ions released into the aqueous environment; the antimicrobial effect is probably based on the release of the Ag⁺ from the silver nanoparticle surface).

The structure of the PU fibres with immobilized molecules of hyperbranched polyethylenimine and consequently immobilized silver nanoparticles on the substrate surface proved to exhibit antimicrobial efficiency against model organisms of strains of *Staphylococcus* and *Escherichia* (tested according to the norm JIS1902/IS020743 for textiles). The tested samples were 5x5 cm in size and the concentration of the inoculum was 10⁵ CFU/mL. The samples functionalized with silver nanoparticles proved to supress the growth of bacterial colonies completely in contrast to the blank samples (the PU fibres without functionalization).

### Example 2: Fibres of poly(lactide-co-glicolide) (PLGA) with immobilized silver nanoparticles on all surfaces.

Nanofibrous structures of poly(lactide-co-glycolide) (PLGA), i.e. fibres of PLGA with the average diameter of hundreds of nanometers are prepared via electrospinning from the mixture of PLGA in chloroform (CHCl₃, 95%) with the addition of dimethylformamide (DMF; 5%). PLGA with the molecular weight of 50,000 - 75,000 g.mol⁻¹ is dissolved in the mixture of chloroform and DMF in the weight ratio of 30%. After homogenization of the mixture of PLGA:DMF:CHCl₃ on a shaker, hyperbranched polyethylenimine (PEI) with molecular weight 25,000 g.mol⁻¹ was added in the concentration of 0.1 wt.% (in the relation to the PLGA mass). (Note: The hyperbranched polymer linker contains numerous nitrogen atoms with free electron pair, which is used for the reduction, stabilization and immobilization of the generated silver particles.) The resulting mixture is again homogenized on a shaker. Subsequently, oriented fibres are prepared via electrospinning and collected on a rotation collector. This way prepared fibres, containing polymer linker in its structure for targeted deposition of silver nanoparticles, are consequently immersed into the solution of silver nanoparticle precursor, i.e. into silver nitrate with the concentration equal to 5·10⁻³ mol.L⁻¹, for the period of 3 minutes. Then they are taken out and washed with distilled water in order to eliminate the chemical residues, especially traces of silver nitrate as the nanoparticle precursor. There is a strong coordination-covalent interaction between the heteroatoms and silver. The linker itself is firmly anchored in the structure of the fibre, which prevents release of either silver nanoparticles or silver nanoparticles with the polymer linker.

### Example 3: Nanofibres of polylactide-co-glycolide (PLGA) with immobilized silver nanoparticles on all fibre surfaces.

Nanofibrous structures of polylactide-co-glykolide (PLGA), i.e. fibres with the diameter of tents of nanometers are prepared via electrospinning from the mixture of PLGA dissolved in tetrahydrofuran (THF, 50%) and chloroform (CHCl₃, 50%). PLGA (with the molecular weight of 50,000 - 75,000 g.mol⁻¹) is dissolved in the mixture of chloroform and THF in the weight ratio of 30%. After homogenization of the mixture of PLGA:THF:CHCl₃ on a shaker, hypebranched polymer linker - polyethylenimine is added to the mixture (the molecular weight of polyethylenimine is 750,000 g.mol⁻¹; note: the hyperbranched polymer linker contains sufficient number of nitrogen atoms with free electron pair, which is used in the process of reduction, stabilization and immobilization of silver nanoparticles)) in the concentration of 0.1 wt.% (in the relation to the PLGA mass). Subsequently, the fibres were prepared via electrospinning. The fibres were collected on a rotation collector without any orientation - in a form called "mesh". This way prepared fibres, involving polymer linker in its structure for targeted deposition of silver nanoparticles, are consequently immersed into the solution of silver nanoparticle precursor, i.e. into silver nitrate with the concentration equal to 1.10⁻² mol.L⁻¹, for the period of 3 minutes. Then they are taken out and properly washed with distilled water in order to eliminate the chemical residues, especially the any traces of the silver nitrate as the precursor. There is a strong coordination-covalent interaction between the heteroatoms in polymer linker structure and silver. The linker itself is firmly anchored in the structure of the fibre, which prevents release of either silver nanoparticles or silver nanoparticles with the polymer linker.

## Claims

1. A method of preparation of a polymer substrate with immobilized silver nanoparticles, **characterized in that** the substrate is prepared via electrospinning of a mixture of water non-soluble polymer and a polymer linker containing Ag-reducing functional groups containing nitrogen atom having a free electron pair, selected from the group comprising amino-, imino-, imido groups, and consequently, the substrate is subjected to aqueous solution of silver ions resulting in formation of silver nanoparticles covalently immobilised to the substrate via nitrogen atom of the polymer linker.

2. The method according the claim 1, **characterized in that** the water non-soluble polymer is a synthetic polymer, preferably selected from a group comprising polyurethanes, polylactides, polyglycolides, poly(lactide-co-glycolide), polyethylene, polypropylene, polyamide, saccharide, as e.g. cellulose or chitosan.

3. The method according to any one of the preceding claims, **characterized in that** the polymer linker is polyethylenimine.

4. The method according to any one of the preceding claims, **characterized in that** the polymer linker is contained in the substrate in the concentration range from 0.0001 wt. % to 5 wt. %, related to the overall mass of the water non-soluble polymer.

5. The method according to any one of the preceding claims, **characterized in that** the silver ions are in a form of a water soluble silver salt or a water soluble complex of silver ions.

6. The method according to any one of the preceding claims, **characterized in that** the application of the aqueous solution of silver ions on the substrate is performed at the concentration of the silver ions in the range of from 0.0001 mol·L⁻¹ to 5 mol·L⁻¹; preferably at the temperature of the media from 5 °C to 95 °C, preferably for the time period from 1 s to 60 minutes.

7. Antimicrobial material with the content of silver nanoparticles, **characterized in that** it contains nanofibrous substrate from a mixture of water non-soluble polymer and a polymer linker containing functional groups comprising nitrogen atoms with free electron pairs, selected from the group comprising amino-, imino-, imido groups, wherein silver nanoparticles are covalently immobilized to those functional groups, wherein the antimicrobial material is formed by a method of any of the preceding claims.

## Patentansprüche

1. Verfahren zur Herstellung eines Polymersubstrats mit immobilisierten Silbernanopartikeln, **dadurch gekennzeichnet, dass** das Substrat durch Elektrospinnen eines Gemisches aus wasserunlöslichem Polymer und einem Polymerlinker hergestellt wird, der Ag-reduzierende funktionelle Gruppen enthält, die ein Stickstoffatom mit einem freien Elektronpaar, wobei die Gruppen ausgewählt aus der Gruppe bestehend aus Amino-, Imino-, Imidogruppen sind, und folglich wird das Substrat einer wässrigen Lösung von Silberionen ausgesetzt, was zur Bildung von Silbernanopartikeln führt, die über ein Stickstoffatom des Polymerlinkers kovalent an dem Substrat immobilisiert sind.

2. Verfahren nach Anspruch 1, **dadurch gekennzeichnet, dass** das wasserunlösliche Polymer ein synthetisches Polymer ist, vorzugsweise ausgewählt aus einer Gruppe bestehend aus Polyurethanen, Polylactiden, Polyglycoliden, Poly(lactid-co-glycolid), Polyethylen, Polypropylen, Polyamid, Saccharid, wie zum Beispiel Cellulose oder Chitosan.

3. Verfahren nach einem der vorhergehenden Ansprüche, **dadurch gekennzeichnet, dass** der Polymerlinker Polyethylenimin ist.

4. Verfahren nach einem der vorhergehenden Ansprüche, **dadurch gekennzeichnet, dass** der Polymerlinker im Substrat im Konzentrationsbereich von 0,0001 Gew.% bis 5 Gew.% enthalten ist, bezogen auf die Gesamtmasse des wasserunlöslichen Polymers.

5. Verfahren nach einem der vorhergehenden Ansprüche, **dadurch gekennzeichnet, dass** die Silberionen in Form eines wasserlöslichen Silbersalzes oder eines wasserlöslichen Komplexes von Silberionen vorliegen.

6. Verfahren nach einem der vorhergehenden Ansprüche, **dadurch gekennzeichnet, dass** das Aufbringen der wässrigen Lösung von Silberionen auf das Substrat bei einer Konzentration der Silberionen im Bereich von 0,0001 mol.L⁻¹ bis 5 mol.L⁻¹; vorzugsweise bei der Temperatur des Mediums von 5 °C bis 95 °C, vorzugsweise für den Zeitraum von 1 s bis 60 Minuten.

7. Antimikrobielles Material mit dem Gehalt an Silbernanopartikeln, **dadurch gekennzeichnet, dass** es nanofasriges Substrat aus einer Mischung von wasserunlöslichem Polymer und einem Polymerlinker enthält, der funktionelle Gruppen mit Stickstoffatomen mit freien Elektronenpaaren enthält, ausgewählt aus der Gruppe bestehend aus Amino-, Imino-, Imidogruppen, wobei Silbernanopartikel kovalent an jene funktionellen Gruppen immobilisiert sind, wobei das antimikrobielle Material durch ein Verfahren nach einem der vorhergehenden Ansprüche hergestellt wird.

## Revendications

1. Procédé de préparation d'un substrat polymère avec des nanoparticules d'argent immobilisées, **caractérisé en ce que** le substrat est préparé par électrofilage d'un mélange de polymère non soluble dans l'eau et d'un lieur polymère contenant des groupes fonctionnels réducteurs d'Ag contenant un atome d'azote à paire libre d'électrons, lesdits groupes étant choisis dans le groupe comprenant les groupes amino, imino, imido, et puis le substrat est contacté avec une solution aqueuse d'ions argent, ce qui entraîne la formation de nanoparticules d'argent immobilisées de manière covalente sur le substrat via l'atome d'azote du lieur polymère.

2. Procédé selon la revendication 1, **caractérisé en ce que** le polymère non soluble dans l'eau est un polymère synthétique, de préférence choisi dans un groupe comprenant les polyuréthannes, les polylactides, les polyglycolides, le poly (lactide-co-glycolide), le polyéthylène, le polypropylène, le polyamide, le saccharide, comme par exemple cellulose ou chitosane.

3. Procédé selon l'une quelconque des revendications précédentes, **caractérisé en ce que** le lieur polymère est la polyéthylène imine.

4. Procédé selon l'une quelconque des revendications précédentes, **caractérisé en ce que** le lieur polymère est contenu dans le substrat dans la gamme de concentration allant de 0,0001% en poids à 5% en poids, rapporté à la masse totale du polymère non soluble dans l'eau.

5. Procédé selon l'une quelconque des revendications précédentes, **caractérisé en ce que** les ions argent sont sous la forme d'un sel d'argent soluble dans l'eau ou d'un complexe d'ions d'argent soluble dans l'eau d'ions d'argent.

6. Procédé selon l'une quelconque des revendications précédentes, **caractérisé en ce que** l'application de la solution aqueuse d'ions argent sur le substrat est effectuée à la concentration des ions argent dans la gamme de 0,0001 mol.L⁻¹ à 5 mol.L⁻¹; de préférence à la température du solvant de 5 °C à 95 °C, de préférence pendant la durée de 1 seconde à 60 minutes.

7. Matériau antimicrobien aven des nanoparticules d'argent, **caractérisé en ce qu'**il contient un substrat nanofibreux composé d'un mélange de polymère non soluble dans l'eau et d'un lieur polymère contenant des groupes fonctionnels comprenant des atomes d'azote avec des paires d'électrons libres, lesdits groupes étant choisis dans le groupe comprenant les groupes amino, imino, imido, dans lesquels des nanoparticules d'argent sont immobilisées de manière covalente sur ces groupes fonctionnels, où le matériau antimicrobien est formé par un procédé selon l'une quelconque des revendications précédentes.
